# EUROPEAN PATENT APPLICATION

(11) **EP 4 335 386 A1**
(43) Date of publication of application: **13.03.2024**
(21) Application number: 22315207.5
(22) Date of filing: 09.09.2022
(51) Int. Cl.: A61B 17/00

(54) **OCCLUDING DEVICE FOR CLOSING A WOUND IN A PATIENT**

(71) Applicant: Caranx Medical SAS, 75013 Paris (FR)
(72) Inventor: SEJOR, Eric, 06000 Nice (FR); CERRUTI, Giulio, 06700 Saint-Laurent-Du-Var (FR); SMITS, Jonas Victor Harmen, 3360 Bierbeek (BE); BERTHET-RAYNE, Pierre, 06800 Cagnes-sur-Mer (FR)
(74) Representative: Hepp Wenger Ryffel AG

(57) **Abstract**

An occluding device (10) adapted for closing a wound in a tissue wall of a patient comprises a tubular membrane (20) having interconnected first and second extremities (20a, 20b). At least one extremity comprises an expandable annular structure (30) for affixing a surface of a tissue wall of a patient. A through-lumen (21) of the tubular membrane (20) is closable by torsion.

## Description

The present invention relates to an apparatus and a method of use for closing a wound in a patient according to the independent claims.

Wounds, whether intentional or non-intentional, can affect more than just the top layers of the skin and reach deeper tissues such as vessels. This type of wound often requires immediate attention to prevent haemorrhaging of the patient.

Various techniques and devices have been developed to assist in closing wounds in patients. The choice of device often varies depending on the type; size and severity of the wound.

Most superficial injuries can be treated using stiches, staples and even skin glue. More serious injuries call for more invasive devices to ensure closing of the wound.

In Patent US 2019/336114 A1 a closure device is provided for closing the carotid artery. The closure device has a first disk to be delivered to a first vessel and a second disk for delivery to a second vessel that is adjacent to the first vessel. The device further comprises a compression element for pulling the pulling the first and second disks together. This closure device is, however, relatively complicated in its use and construction.

Devices of the prior art are generally difficult and time consuming to manufacture and/or deliver and can result in deployment failure or are take too long to deploy to be able to be used in emergent situations.

Additionally, most wound closing devices are adapted to close either superficial wounds or close subcutaneous the vessel wound and rarely adapted to entirely close deep wounds. Further drawbacks lie in the delivery and deployment of devices adapted for deep wounds wherein multiple instruments and substantial manipulation is required.

It is an object of the present invention to overcome the drawbacks of the prior art and in particular to provide a device that shall allow easy and fast closing of wounds or incisions formed in a tissue or vessel.

The occluding device according to the invention is adapted for temporarily closing an opening in a tissue wall of a patient. The opening in a tissue wall may be for example a wound or a surgical access site.

A wound is generally understood to be an injury, accidental or intentional, wherein to at least one layer of tissue is broken/opened.

The wound may extend from the skin of the patient toward a deeper layer of tissue, e.g. to a vessel.

The occluding device comprises a tubular membrane having interconnected first and second extremities. At least one extremity comprises an expandable annular structure for affixing a surface of a tissue wall of a patient.

Optionally both extremities may be provided with a radially expandable annular structure.

The annular structure may be affixed to the tissue wall in such a manner that the annular structure may be moveable with regards to the tissue wall, e.g. rotate. Additionally or alternatively, one or more annular structures may be affixed to the tissue wall in such a manner that the annular structure is fixedly in contact with the tissue wall.

Affixing the annular structure to the tissue wall may avoid the displacement and migration of the device within the patient.

The device, more specifically the tubular membrane, further has a through-lumen. The through-lumen of the tubular membrane may be closable by torsion of one extremity of the tubular membrane with respect to the other extremity.

Torsion of the tubular membrane may be achieved by rotating one of the extremities of the tubular membrane. Rotation of one or more of the extremities will induce a twisting of the tubular membrane on itself. This reduces the size of the through-lumen by creating a waist situated between the two extremities. For example, the waist may be equidistant from the first and second extremity.

Such an occluding device may allow for an easier and quicker closing of wounds in emergent situations. It can additionally allow for a temporary closing of the wound that is re-openable, e.g. during a procedure, and thus prevent unnecessary blood loss.

The device or components of the device may be made of an at least partly elastic material, preferably a biocompatible elastic material.

Optionally, the device may be made of a resorbable biocompatible material. This allows for a temporary device that is resorbed after a certain period of time.

The annular structure is adapted to be brought sealingly in contact with the opening of the tissue wall of the patient.

In other words the annular structure is adapted such that when it is in contact with the tissue wall, the passage and/or leakage of a liquid through a gap between the annular structure and the tissue and hence through the opening within the tissue wall is reduced or prevented. For example, when used to close a vessel wound, blood is prevented from leaking through the contact site between the annular structure and the tissue wall.

The sealing contact between the annular structure and the opening in the tissue helps reduce the risk of blood leakage and may also reduce the risk of pathogens passing through the opening.

The device and in particular the annular structure is preferably configured to radially expand from a delivery configuration to a deployed configuration.

In the delivery configuration the device, particularly the annular structure, is compressed in such a manner that the device, and preferably the annular structure, is at least partly smaller than in the deployed configuration. This allows for easier delivery of the device within a patient and may contribute to reducing the invasiveness of the procedure.

In the deployed configuration the annular structure is expanded so that it can get in contact with the tissue wall near the opening in the tissue wall.

Optionally, the annular structure in the deployed configuration at least partly and preferably fully surrounds the opening in the tissue wall.

The expansion of the annular structure from the delivery configuration to the deployed configuration may induce the sealing contact between the annular structure and the opening in the tissue.

The annular structure may comprise or receive at least one expansion element for expanding the annular structure.

The expansion of the annular structure may be induced with an instrument, e.g. an a guide wire or an inflatable balloon, which will form the expansion element.

Alternatively, the annular structure may be self-expandable. For example, the annular structure may be made of an elastic material and therefore expand due to elasticity, which will form the expansion element.

The annular structure may in particular house at least one self-expandable expansion element for expanding the annular structure, e.g. a spring.

A self-expandable annular structure reduces the need for external devices or instruments to expand the annular structure and thus facilitates a fast and easy closing of the wound.

Alternatively or additionally, the annular structure may comprise an inner cavity for receiving an expansion element in the form of an expansion fluid for expanding the annular structure.

The inner cavity may be in the form of an internal compartment adapted to accommodate an expansion fluid and to expand upon receiving an expansion fluid.

The tubular membrane may further comprise at least one channel for guiding an expansion fluid to the annular structure, thus inducing the expansion of the annular structure.

The channel of the tubular membrane may extend longitudinally along at least a part of the tubular membrane.

The channel of the tubular membrane and/or the inner cavity of the annular structure may be leak-proof compartments for avoiding leakage of the expansion fluid.

In some embodiments the annular structure and at least one channel of the tubular membrane may be connected for expanding the occluding device from a delivery configuration to a deployed configuration. Preferably, the annular structure and at least one channel of the tubular membrane may be connected for expanding the annular structure of the device.

Optionally the at least one channel may comprise a connector for fluidly connecting the channel to a fluid supply.

A fluidly expandable annular structure is preferably provided in a semi-permanent and/or permanent occluding device configured to be removed from the patient after a period of time.

The expansion element may be at least partly elastic. Additionally, the expansion element may be configured to transition from a first state to a second state for expanding the device.

The transition of the expansion element may be induced by an instrument, e.g. a guide-wire. Additionally or alternatively, the expansion element may self-expand from a first state to a second state.

The expansion element may be any suitable element adapted to expand and/or self-expand. For example, the expansion element may be a preloaded spring or may be made of an elastic or memory shape material.

In some embodiments at least the first extremity of the occluding device may be configured to be rotatable with regards to the second extremity of the occluding device. The rotation of at least the first extremity may induce the torsion of the occluding device so that the cross section of the through-lumen of the tubular membrane is reduced in at least one waist point for closing the through-lumen.

In some embodiments the first and second extremities may both be configured to rotate, in particular in opposite directions with respect of each other to induce the closing of the through-lumen.

The rotation of one or more extremities reduces the diameter of the through-lumen at the waist point. In a fully occluding state, the through-lumen of the device is fully closed at the waist point and thus prevents the passage of fluids through the through-lumen.

In some embodiments at least one of the annular structure, or parts thereof, may be configured to be rotatable to induce the closing of the opening in a tissue wall of a patient.

Rotation of the annular structure may induce the torsion of the connected tubular membrane and thus the closing of the though-lumen at the waist point.

The invention may further comprise a valve. Preferably the valve may be positioned at least partly near an extremity of the tubular body.

Providing the device with a valve may help maintain a surgical access site open whilst avoiding unnecessary blood-loss during a procedure.

In some embodiments at least one extremity may attach to or comprise a stent, preferably a vascular stent. Optionally the annular structure may attach to the stent.

The occluding device may be formed as a permanent occluding device for closing an opening in a tissue wall of a patient. Alternatively, the occluding device may be a temporary occluding device for closing an opening in a tissue wall of a patient.

The device may be adapted to provide a temporary closure of the wound, for example during a procedure when access may be needed at a later time.

It may also be used as a more permanent closing of the wound, for example, the device can be placed until such time that homeostasis is reached around the waist point of the device. Once homeostasis is reached the device may be removed. Optionally, the device may resorb during the healing process of the wound.

The occluding device, and optionally the annular structure, in the expanded state are sized and shaped to close an opening in a cutaneous wound. Additionally or alternatively the occluding device and preferably the annular structure, in the expanded state may be sized and shaped to close an opening in a vascular tissue wall of a patient. Typically, in the expanded state, the device has a length of 1 mm to 20 mm and/or a diameter of 1 mm to 10 mm, preferably of 3 mm to 5 mm.

In a second aspect the invention may provide a method of closing an opening in a tissue wall of a patient. Optionally an occluding device as described above is used.

An occluding device is introduced within an opening of the tissue wall, e.g. a wound, of the patient.

The occluding device is then expanded within or neighbouring a tissue, optionally near an opening in the tissue wall of the patient. The device may be expanded in a manner such that the occluding device completely surrounds the opening in the tissue wall.

The occluding device is then torsioned or twisted to close an opening in the tissue wall of the patient. Optionally, one or both extremities of the device are torsioned to occlude the opening in the tissue wall.

The occluding device may remain in the patient until homeostasis is nearly reached. The method hence may comprise a further step of removing the occluding device once homeostasis is nearly reached. For removal, the occluding device can be brought back into a compressed configuration, similar or identical to the initial delivery configuration. This can be achieved by reversing an expansion mechanism, e.g. by removing an expansion fluid from fluidly expandable annular structures.

The invention will be better understood with reference to the following decryption of preferred embodiments and the accompanying drawings, which show:
- Fig. 1: a schematic side view of an embodiment of an endoluminal device for closing an opening in a tubular structure of a patient,
- Fig. 2: a schematic side view of a second embodiment of an endoluminal device for closing an opening in a tubular structure of a patient,
- Fig. 3: a schematic side view of a third embodiment of an endoluminal device for closing an opening in a tubular structure of a patient,
- Figs. 4a-b: schematic front and side views of a valve of an endoluminal device for closing an opening in a tubular structure of a patient,
- Fig. 5: a schematic side view of a fourth embodiment of an endoluminal device for closing an opening in a tubular structure of a patient,
- Fig. 6a-b: schematic front views of an embodiment of an expansion element of an endoluminal device for closing an opening in a tubular structure of a patient,
- Figs. 7a-d: schematic diagrams illustrating steps in an example technique for closing an opening in a tubular structure of a patient with an endoluminal device according to the invention,
- Figs. 8a-d: schematic diagrams illustrating steps of an alternative technique for closing an opening in a tubular structure of a patient
- Figs. 9a-b: schematic side views of a fifth embodiment of an endoluminal device for closing an opening in a tubular structure of a patient
- Fig. 10a-c: schematic views of another embodiment of an endoluminal device for closing an opening according to the intention and
- Fig. 11: a delivery device for the embodiment of Fig. 10a to 10c.

In the illustrations, the same reference numerals are used to denote the same or equivalent features amongst different embodiments and examples. Unless described to the contrary, the de-scription of a feature in one embodiment may also apply to the same or equivalent feature in one embodiment or example. Features may also be interchanged in embodiments as desired.

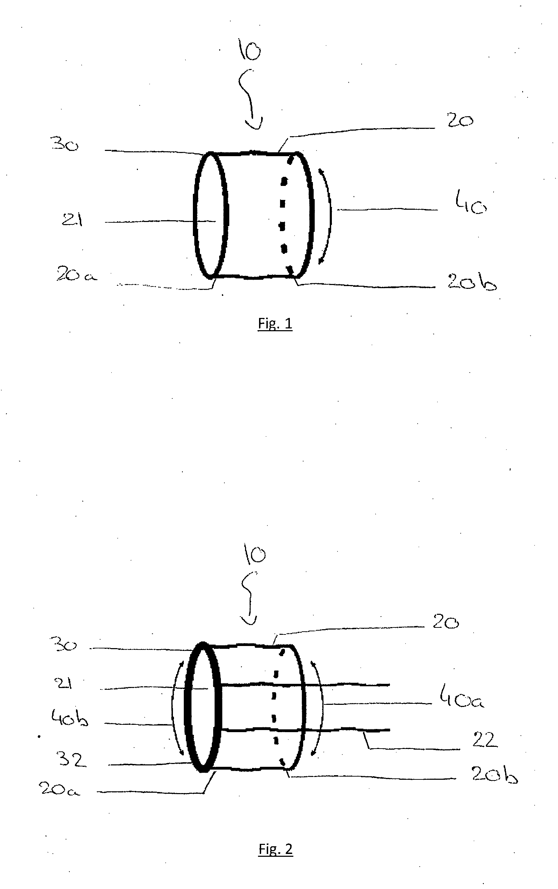

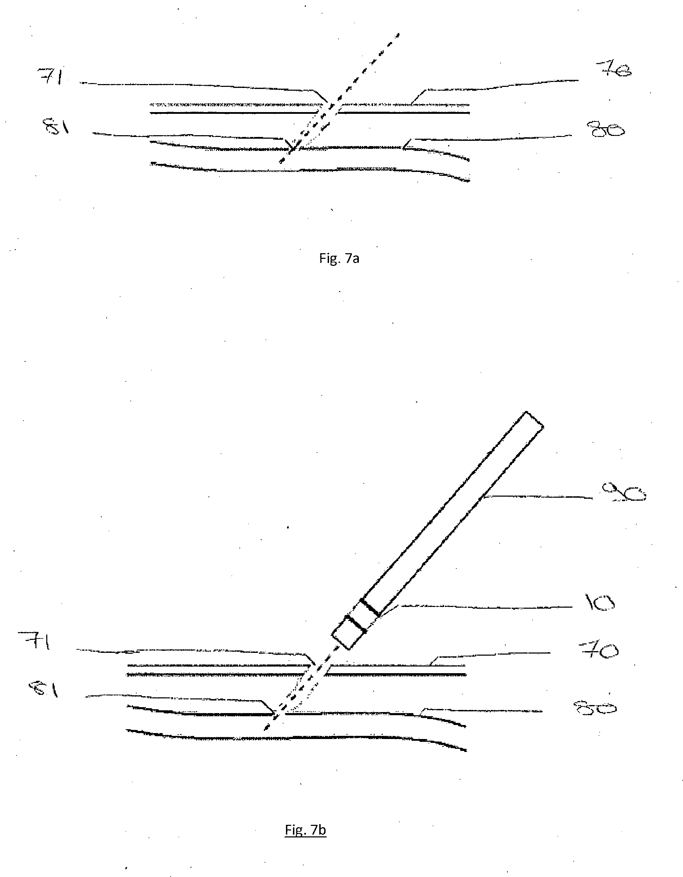

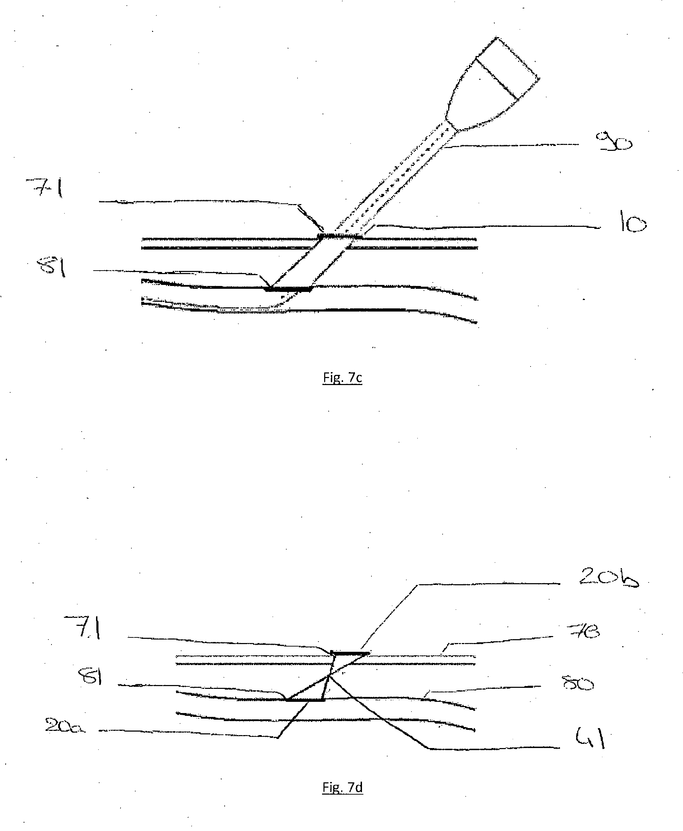

Referring to figure 1 an occluding device 10 comprises a tubular membrane 20. The tubular membrane has a first extremity 20a and second extremity 20b and further defines a through-lumen 21 extending between the first and second extremity 20a and 20b. The device 10 further has an annular structure 30 at the first extremity 20a of the tubular membrane 20. At least one of the extremities 20a or 20b is adapted to rotate in direction 40 around a longitudinal axis with regards to the other extremity. The rotation 40 of at least one extremity 20a or 20b induces a torsion of the device 10. Torsion of the device closes the through-lumen 21 of the tubular membrane 20 at a waist point 41 (see Fig. 7d) and thus occludes an opening 71 or 81 in a tissue wall 70, 80 of a patient (see figs. 7-8).

Shown in figure 2, an alternative occluding device 10 comprises a tubular membrane 20 extending between a first extremity 20a and a second extremity 20b. The occluding device 10 has a through-lumen 21 defined by a tubular membrane 20 and an annular structure 30.

The annular structure 30 is expandable to occlude an opening of a tissue wall of a patient. The annular structure 30 is configured to expand from a delivery configuration to a deployed configuration. To this purpose the annular structure 30 has an inner cavity 32 adapted to receive an expansion element. In this embodiment, the expansion element is an expansion fluid (not shown).

The tubular membrane 20 of the device 10 has a plurality of channels 22 extending along its length. The channels 22 are fluidly connected with a cavity within the annular structure 30 and can guide an expansion fluid to the annular structure 30 allowing the expansion of the annular structure from a delivery configuration to a deployed configuration.

The first and second extremities 20a and 20b, of the tubular membrane 20 are configured to rotate, preferably in opposite directions 40a and 40b, with regards to one another. Rotating the extremities 20a and 20b in opposing direction 40a and 40b induces a torsion of the tubular membrane 20 and thus occludes the through-lumen 21 of the device 10 at the waist point 41 (see Fig. 7d).

The channels 22 can have a connector (not shown) for connecting to a fluid supply (not shown) for providing an expansion fluid (e.g. a saline solution, but also a gas) to expand the annular structure 30.

Optionally the twisting and hence the occluding of the device 10 can be induced by the rotation 40 of the annular structure 30 and/or by an additional annular structure at extremity 20b, with regards to the other extremity or the other annular structure, respectively.

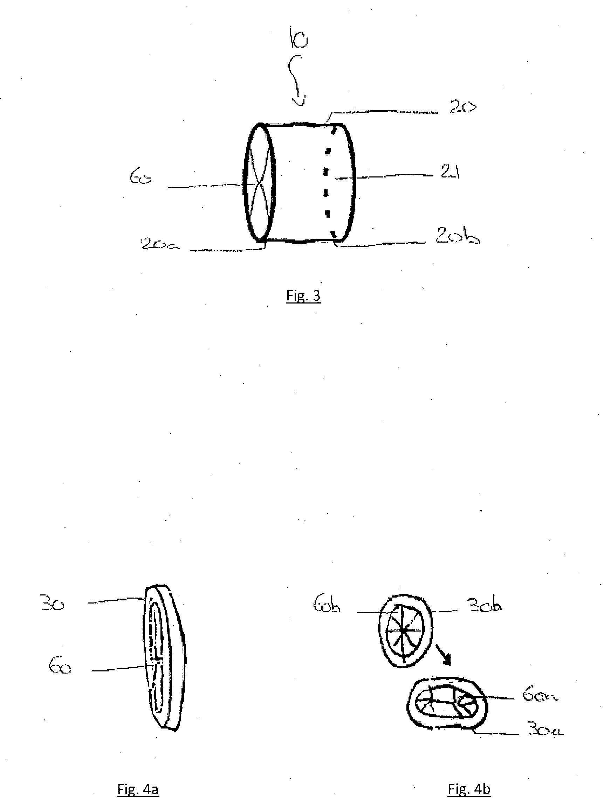

Illustrated in figure 3, another embodiment of an occluding device 10 has a through-lumen 21 defined by a tubular membrane 20. The tubular membrane has a first extremity 20a and a second extremity 20. The device 10 further comprises a valve 60 positioned at a first extremity 20a of the tubular membrane 20.

Seen in figure 4a and 4b the valve 60 can be attached to an annular structure 30 of a device 10 for occluding an opening in a tissue wall of a patient.

The annular structure 30 and the optional valve 60 are at least partly malleable/flexible. This allows the annular structure, and when provided the valve 60, to expand from a radially compressed delivery configuration 30b and 60b (upper .schematic representation in Fig. 4b) to an expanded deployed configuration 30a and 60a (lower schematic representation in Fig. 4b).

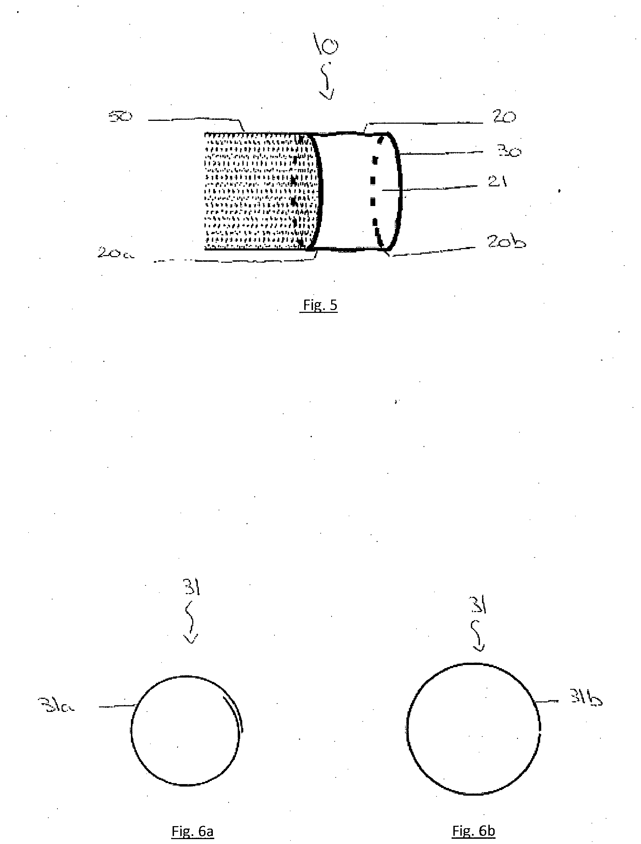

Referring to figure 5, still another embodiment of an occluding device 10 comprises a tubular membrane 20 having through lumen 21. The tubular structure 20 extends from a first extremity 20a and a second extremity 20b. The device 10 further comprises an annular structure in the form of a stent 50 attached to a first extremity 20a of the tubular membrane 20. An annular structure 30 is provided at the second extremity 20b of the tubular membrane 20.

Figures 6a and 6b show an exemplary expansion element 31. The expansion element is an elastic ring configured to self-expand from a compressed delivery configuration 31a to an expanded deployed configuration 31b. The elastic ring is housed within an annular structure 30 (see figures 1-5) of the device 10.

Figures 7a-d depict a method for closing a tissue wound in a patient with an occluding device 10. The wound extends along a direction represented by a dashed line from an opening 71 in the top layer of the patient's skin 70 to an opening 81 in subcutaneous layers of tissue, here a vessel 80.

As shown in Fig. 7b, an instrument 90, e.g an introducer sheath, is provided with the occluding device 10 in a compressed delivery configuration for introduction within the patient. The instrument 90 is inserted within the patient through the openings 71, 81. This allows the positioning of the device 10. The introducer sheath and the device retained therein in a compressed configuration may be e.g. formed in a manner known from devices which a insertable in a minimally invasive manner, such as e.g. stents or heart valves.

The device 10 is deployed from the sheath in a manner that a first extremity 20a (see figs. 1-3 and 5) is sealingly in contact with the opening 81 in the vessel 80 and a second extremity 20b of the device 10 is sealingly in contact with the opening 71 in the skin 70 (see Fig. 7c).

The device 10 is operated to induce a torsion of the device in one point, e.g. by rotation of the extremity 20b (see Fig. 7d). The torsion of the device creates a waist point 41 closing off the through-lumen on the device 10. The torsion can be induced by manually rotating the instrument before releasing the extremity 20b.

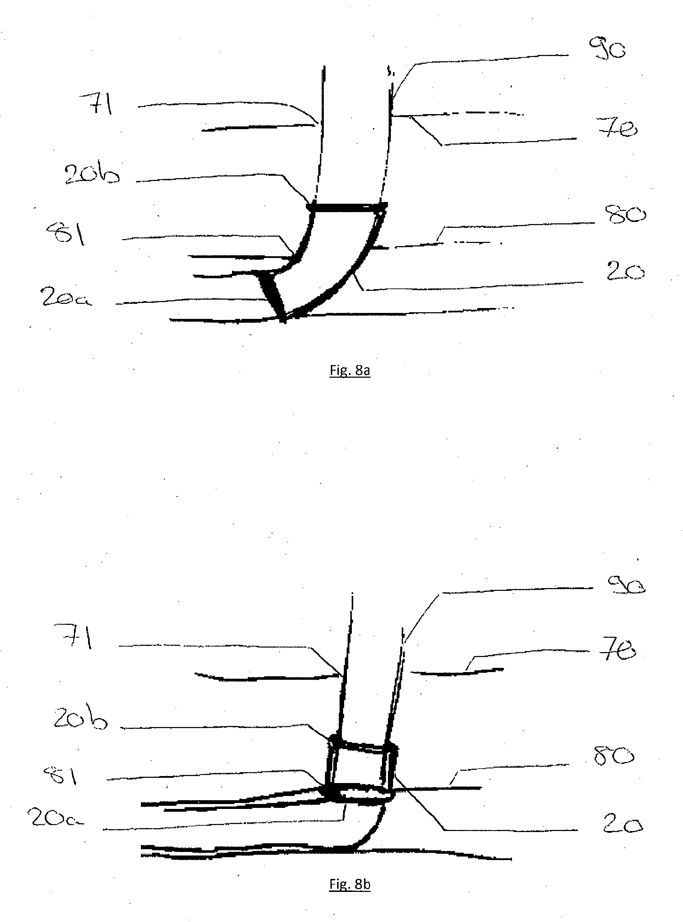

Figures 8a-d demonstrate an alternative method of closing the wound represented in Figs 7a to 7d with an alternative occluding device 10. The device 10 is delivered in a compressed delivery configuration to the opening 81 in subcutaneous tissue 80, in the vessel, by an instrument 90 (Fig. 8a).

The device 10 is deployed to a deployed configuration within the opening 81 (see Fig. 8b).

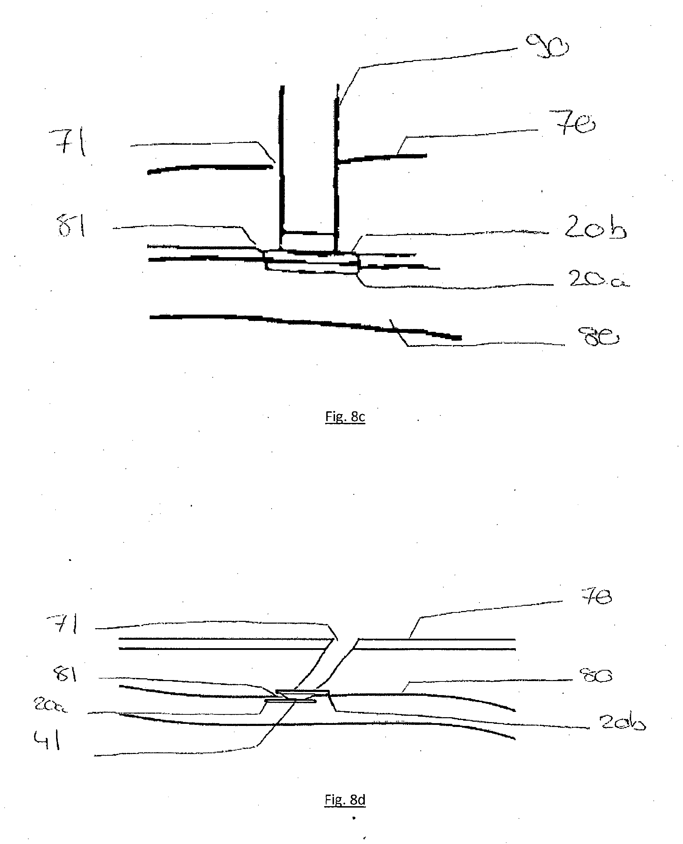

A first extremity 20a of the device 10 is positioned on an inner surface of the vessel 80 at the opening 81. A second extremity 20b is positioned on an external surface of the vessel 80 at the opening 81 (see Fig. 8c).

The device 10 is operated to torsion the device 10 creating a waist point 41 of the through-lumen and thus closing the subcutaneous opening 81 in tissue 80 (Fig. 8d).

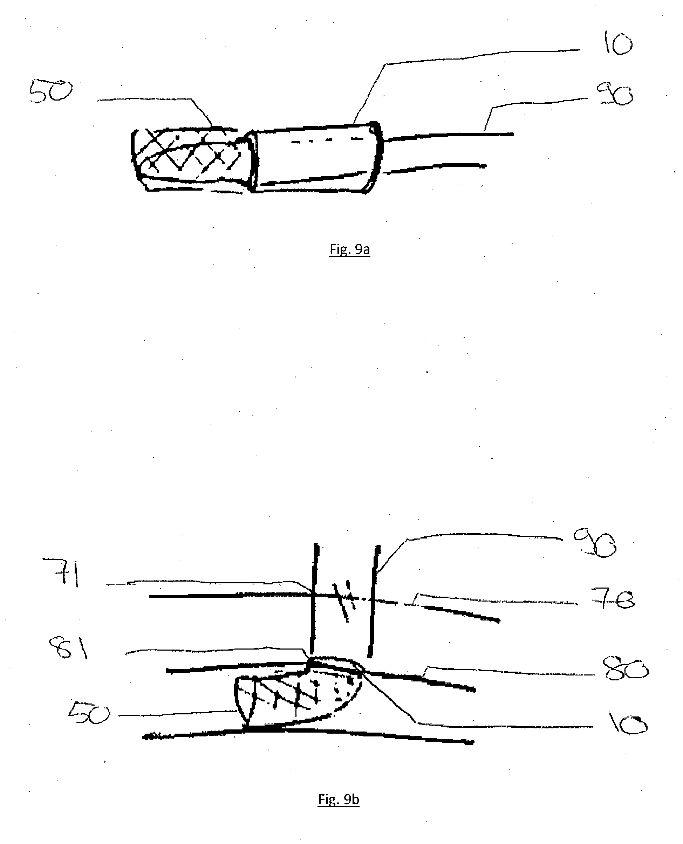

Figures 9a-b show the closing of a wound with an occluding device 10 comprising a stent 50. The instrument 90 delivers the device 10 together with the stent 50 within an opening 81 of a patient. The stent 50 is positioned within a vessel 80 and a tubular membrane of the device 10 is positioned at the opening 81 of the vessel 80. A torsion for closing the opening is created in a similar way as in the previous embodiment.

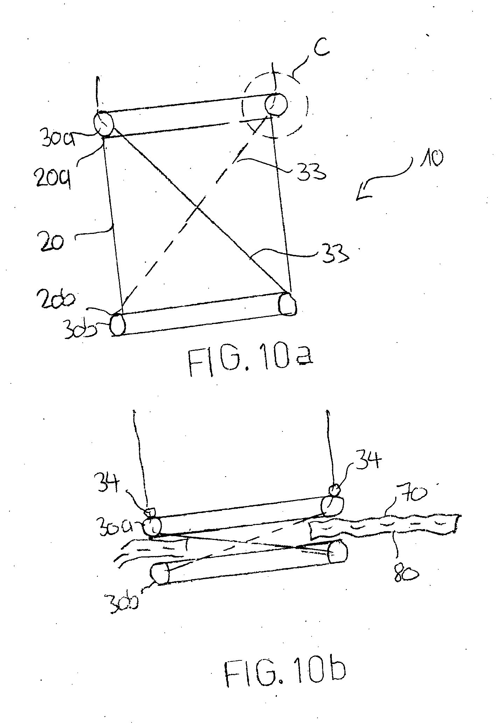

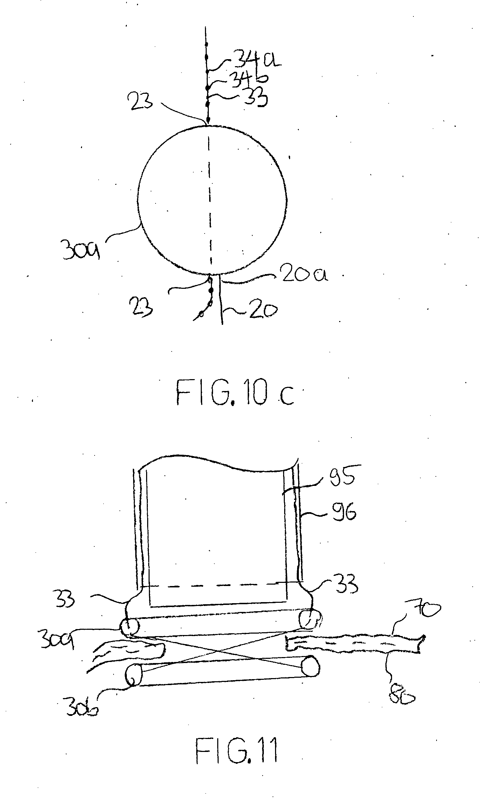

Fig. 10a shows another embodiment of an occluding device 10 according to the invention. Similar as in previous embodiments, the device 10 has a tubular membrane 20 with a first extremity 20a and a second extremity 20b. The extremities 20a, 20b are each connected to a respective annular structure 30a, 30b. The two opposing annular structures 30a, 30b are further linked to each other by sutures or wires 33 which are helically arranged around the membrane 20 such as to run in a circumferential and an axial direction. A first end of the sutures 33 is fixedly attached to one of the annular structures 30b, whereas the other end of the sutures 33 is slidingly guided through the other extremity 30a via openings 23 (see Fig. 10c).

The helical arrangement of the sutures 33 allows to achieve a twisting between the two annular structures 30a, 30b and hence a torsion of the membrane 20, as described above.

Fig. 10b shows the occluding device 10 in a finally deployed position. By pulling the sutures 33, the annular structures 30a, 30b are brought together in an axial direction and are at the same time twisted with respect to each other.

Tissue surfaces 70, 80 are contacted by the respective annular structures 30a, 30b such that the tissue surrounding an opening is tightly engaged and the opening is closed by the torsioned membrane 20. The device is fixed in this position by a knot 34, preventing the sutures 33 from sliding back.

Fig. 10c shows an enlarged view of the section C in Fig. 10a. The sutures 33 are fed into and out of the annular structure 30 via openings 23. Preformed knots 34a, 34b, ... on the sutures 33 allow for an intermediate positioning of the sutures and a step by step pulling, before a final knot 34 (see Fig. 10b) will be formed for fixing the occluding device 10 in its final position, as shown in Fig. 10b.

Fig. 11 shows a device similar to the embodiment of Figs 10a to 10c, with a delivery device. The delivery device has an inner sheath 95 which initially receives the occluding device 10 in a radially compressed configuration. By moving the occluding device out of the distal end of the inner sheath 95, the occluding device 10 can be deployed and expands radially. A concentric outer sheath 96 is provided for guiding the sutures 33. Once the occluding device is deployed from the distal end of the inner sheath 95 by withdrawing the inner sheath 95, the annular structures 30,a, 30b can be pulled together and twisted with respect to each other by pulling on the sutures 33 at the proximal end (not shown ) of the outer sheath 96.

## Claims

1. An occluding device (10) for at least temporarily closing an opening (71, 81) in a tissue wall (70, 80) of a patient, the occluding device (10) comprising a tubular membrane (20) having interconnected first (20a) and second extremities (20b), wherein at least one extremity comprises an expandable annular structure (30), for affixing a surface of a tissue wall (70, 80) of a patient, wherein a through-lumen (21) of the tubular membrane (20) is closable by torsion.

2. Occluding device (10) according to claim 1, wherein the annular structure (30) is sealingly in contact with the opening (71, 81) of the tissue wall (70, 80) of the patient.

3. Occluding device (10) according to claim 1 or 2, wherein the annular structure (30) is .self-expandable and comprises and/or comprises or is adapted to receive at least one expansion element (31) for expanding the annular structure (30) .

4. Occluding device (10) according to any claim 1 to 3,
wherein the annular structure (30) comprises an inner cavity (32) for receiving an expansion fluid for expanding the annular structure (30).

5. Occluding device (10) according to any claim 1 to 4,
wherein the tubular membrane (20) comprises at least one channel (22) for guiding an expansion fluid.

6. Occluding device (10) according to claim 5, wherein the channel (22) extends longitudinally along at least a part of the tubular membrane (20).

7. Occluding device (10) according to any claim 5 or 6,
wherein the annular structure (30) and the at least one channel (22) of the tubular membrane (20) are connected for expanding the occluding device (10) from a delivery configuration to a deployed configuration.

8. Occluding device (10) according to any claim 5 to 7,
wherein the at least one channel (22) comprises a connector for fluidly connecting the channel to a fluid supply.

9. Occluding device (10) according to any claim 3 to 8,
wherein the expansion element (31) is at least partly elastic and is configured to transition from a first state (31a) to a second state (31b) for expanding the device.

10. Occluding device (10) according to any claim 1 to 9,
wherein at least the first extremity (20a, 20b) as configured to be rotateable with regards to the second extremity (20a, 20b) of the occluding device (10) such that the rotation of at least the first extremity (20a, 20b) induces a torsion of the occluding device (10) so that the cross section of the through-lumen (21) of the tubular membrane (20) is reduced in at least one waist point (41) for closing the through-lumen (21).

11. Occluding device (11) according to any claim 1 to 10, wherein the first (20a) and second (20b) extremities are configured to be rotateable in opposite directions (40a, 40b) with respect of each other to induce the closing of the through-lumen (21).

12. Occluding device (10) according to any claim 1 to 11, wherein at least one annular structure (30) is configured to be rotateable to induce the closing an opening (71, 81) in a tissue wall of a patient (70, 80).

13. Occluding device (10) according to any claim 1 to 12, further comprising a valve (60), preferably wherein the valve (60) is positioned at least partly near an extremity (20a, 20b) of the tubular membrane (20).

14. Occluding device (10) according to any claim 1 to 13, wherein at least one extremity (20a, 20b), optionally the annular structure (30), attaches to or comprises a stent (50), preferably a vascular stent.

15. Occluding device (10) according to any claim 1 to 14, wherein the occluding device (10) is a permanent occluding device or a temporary occluding device for closing an opening (71, 81) in a tissue wall (70, 80) of a patient.

16. Occluding device (10) according to any claim 1 to 15, wherein the occluding device (10) and preferably the annular structure (30) in the expanded state are sized and shaped to close an opening (71, 81) in a cutaneous and/or vascular tissue wall (70, 80) of a patient.

17. Method of closing an opening (71, 81) in a tissue wall (70, 80) of a patient, optionally using an occluding device (10) according to any claim 1 to 16, wherein the method comprises the steps of:
(ii) introducing an occluding device (10) within an opening (71, 81) of the tissue wall (70, 80) of the patient;
(ii) expanding the occluding device (10) at, or near, an opening (71, 81) in the tissue wall (70, 80) of the patient;
(iii) torsioning the occluding device (10) to close an opening (71, 81) in the tissue wall (70, 80) of the patient.
